Europäisches Patentamt

(19) European Patent Office

Office européen des brevets

(11) Publication number: **0 448 233 A1**

## EUROPEAN PATENT APPLICATION

(12)

(21) Application number: **91301489.0**

(22) Date of filing: **25.02.91**

(51) Int. Cl.⁵: **A61B 17/58**

(30) Priority: **26.02.90 US 485286**

(43) Date of publication of application:
**25.09.91 Bulletin 91/39**

(84) Designated Contracting States:
**AT BE CH DE DK ES FR GB IT LI NL SE**

(71) Applicant: **SMITH & NEPHEW RICHARDS INC.**
**1450 Brooks Road**
**Memphis, Tennessee 38116 (US)**

(72) Inventor: **Davidson, James A.**
**2573 Windy Oaks Drive**
**Germantown, Tennessee 38138 (US)**
Inventor: **Beals, Neil B.**
**500 S. Greer**
**Memphis, Tennessee 38111 (US)**

(74) Representative: **Cole, William Gwyn**
**Corporate Patents Department Smith and**
**Nephew Research Limited Gilston Park**
**Harlow Essex CM20 2RQ (GB)**

(54) Fatigue resistant orthopaedic implant and method of manufacture.

(57)    An orthopaedic implant (10) having improved fatigue resistant capabilities is provided by the use of cold work expansion of the fixation holes (14) of an implant to plastically deform the implant around the periphery of the hole (14) in a uniform manner. A zone of residual compressive stresses is created which thereby improves the fatigue resistance of the implant in the region of the holes.

EP 0 448 233 A1

FIG. 1.

## FATIGUE RESISTANT ORTHOPAEDIC IMPLANT AND METHOD OF MANUFACTURE

This invention relates to an improved method of manufacturing an orthopaedic implant and to structures resulting therefrom. More particularly, this invention relates to a method for improving the fatigue integrity of fixation holes in orthopaedic implants and structures resulting therefrom.

Orthopaedic implants are subject to repetitive cyclic loading within the patient. Consequently, such implants are designed to resist fatigue. Orthopaedic implants, particularly trauma implants, frequently include holes through which screws and other fasteners pass to attach the implant to the bone. In a cyclic loading environment, however, holes reduce the overall strength of the material since they provide less cross-sectional area to accommodate stresses being transferred to the implant through the bone and also act as stress risers which reduce the ability of the implant to tolerate cyclic or fatigue loading. The problem is particularly acute in trauma implants, such as bone plates, intramedullary nails and compression hip screws since these devices, in effect, stabilise broken bone fragments until healing occurs. Thus, the loading imposed on the bone during normal movements of the patient is immediately transferred to the trauma implant which is then placed under greater stress than a permanent prosthetic implant might be. The situation is aggravated if the bone does not heal as expected. In that case, the implant is required to accommodate not only the greater stresses but also a longer cyclic loading period. Under such conditions, fatigue failure of the trauma implant is more likely.

Historically, the standard remedy to prevent fatigue failure in orthopaedic implants as fixation hole regions has been to increase the thickness of the plate in the hole region. However, it has been noted that fatigue failure of orthopaedic implants occurs in the area of highest stress which, for trauma implants, is typically found at the fixation holes. This characteristic remains true even for more improved fatigue resistant metals, such as cold worked type 316L austenitic stainless steel and titanium-6 AL-4V alloys.

Hence, it is desirable to provide a method for manufacturing orthopaedic implants, particularly trauma implants, wherein the fixation hole regions are more fatigue resistant.

The present invention relates to a method for improving the integrity of fixation holes in orthopaedic implants and the structure resulting therefrom.

In accordance with the present invention there is provided an improved orthopaedic implant comprising:
a biocompatible base member said base member having at least one fixation hole; and
said fixation hole having been expanded beyond the elastic limit of the implant material.

The present invention further provides a method for improving the integrity of holes in orthopaedic implants comprising the steps of:
providing an implant having at least one hole; and
expanding the diameter of the hole beyond the elastic limit of the implant material.

Briefly, each fixation hole is cold worked following fabrication of the orthopaedic metal implant by expanding the hole a predetermined amount, typically at least 2.5% of its initial diameter. Cold working expansion is the process of plastically deforming the work piece. That is, the holes of the work piece are expanded beyond the elastic limit of the material.

Typically, the cold work expansion process is peformed near room temperature which is below the recrystallisation temperature of the base metal. As a result of the plastic deformation in the vicinity of the fixation hole, a high residual compressive stress is introduced into the implant. Thus, the material at the hole region is subjected to a lower level of peak tensile stress during tensile fatigue loading conditions and, therefore, an increase in fatigue strength results. Preferably, the fixation hole is expanded between 2.5% to 6% of its initial diameter, and most preferably between 3.5% to 4.5%.

The present invention also permits the machining of the fixation hole to a final diameter following the cold expansion process which may also result in improved fatigue resistance in the hole region and in the overall performance of the implant.

The improved orthopaedic implant comprises a base member which is manufactured from a biocompatible austenitic stainless steel, a biocompatible titanium-based alloy or a biocompatible cobalt-based alloy. The implant includes at least one fixation hole which has been cold worked by expanding the diameter a predetermined amount, preferably between 2.5% to 6% of its initial diameter. In addition, following cold expansion of the fixation hole, the hole region of the orthopaedic implant may be machined to a final diameter resulting in an improvement in the fatigue resistance of the implant in the hole region.

In order to more fully understand the drawings used in the detailed description of the present invention, a brief description of each drawing is provided.

Figure 1 is an elevation view, partly in cross-section, of a compression hip screw plate which has been installed in the femur bone of the patient.

Figure 2 is an elevation view, partly in cross-section, of an intramedullary nail in the femur.

Figure 3 is a partial cross-sectional view of apparatus which is used to practice the present invention.

Figure 4 is a graph of stress versus strain.

Figure 5 is a graph illustrating the improved performance of the present invention.

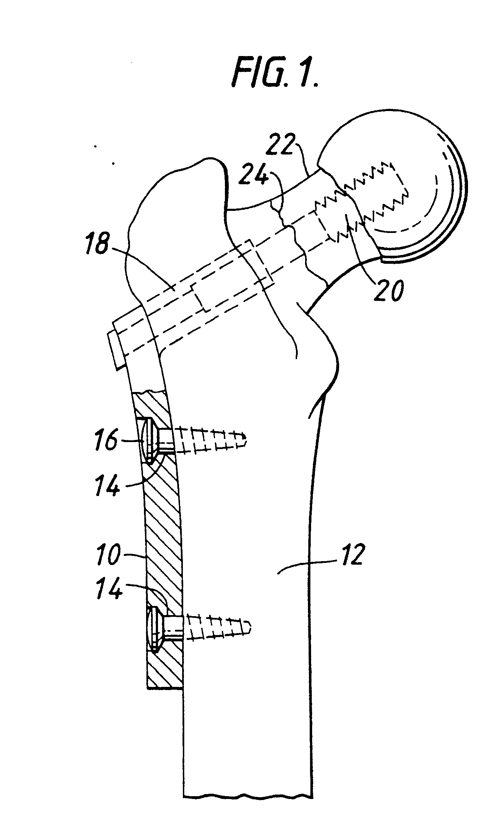

Referring to Figure 1, a compression hip screw plate 10 has been installed in a femur 12. The plate 10 includes at least one fixation hole 14 through which screws 16 and other fasteners pass and attach the plate 10 to the femur bone 12. The plate 10 includes a barrel 18 through which a lag screw 20 passes and is used to affix the femoral head 22 to the upper end of the femur 12. In this manner, body load is transferred from the head 22 to the upper portion of a femur through the lag screw 20 permitting the healing of the fracture 24.

The implant shown in Figure 1 is a trauma device which is installed in an emergency situation and, typically, remains in the patient for a limited period of time such as one or two years. Essentially, this means that the device would be subjected to approximately two million cycles over its term (the average patient normally takes one million steps per year). The present invention as disclosed herein, however, is not limited to application in a trauma implant as shown in Figure 1. It may be incorporated in any number of orthopaedic implants which have at least one fixation hole, or the barrel region of a compression hip screw device.

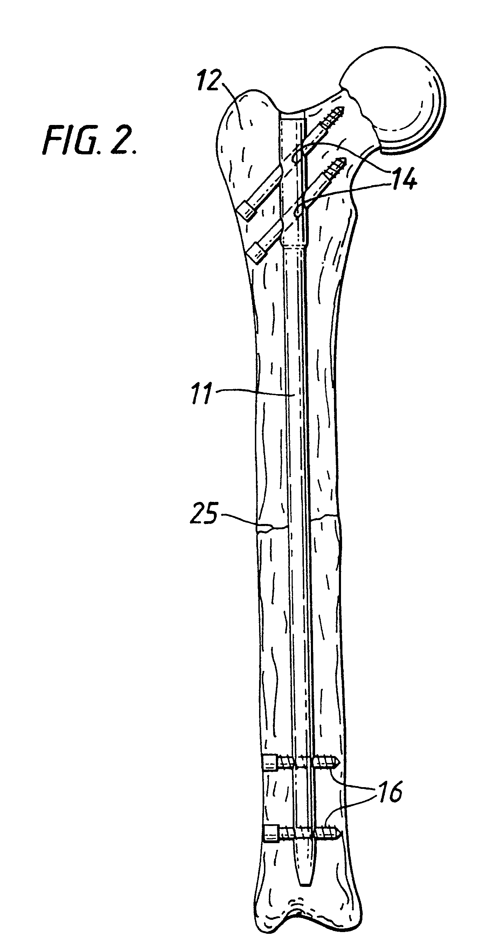

Referring to Figure 2, an intramedullary nail 11 is shown implanted within a femur 12. The intramedullary nail includes fixation holes 14 at both ends. Fasteners or screws 16 are shown passing through the fixation holes 14 at both ends of the intramedullary nail. As shown, the intramedullary nail is a trauma implant used to stabilise a broken femur, for example during the healing process resulting from bone fracture 25.

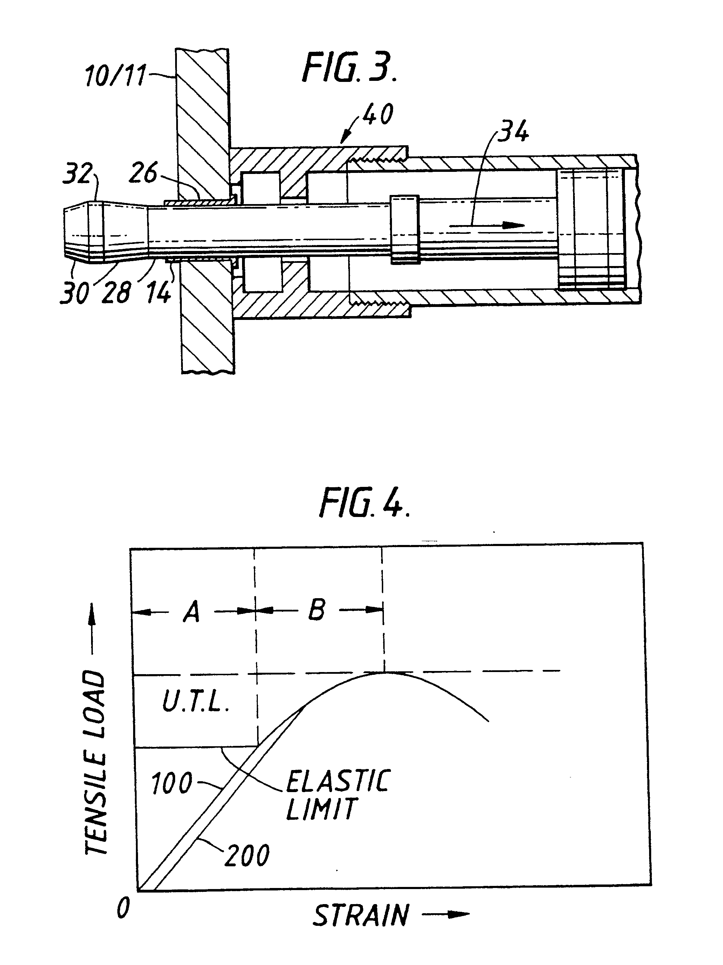

Referring to Figure 3, a prelubricated sleeve 26 is first passed over the end of a mandrel 28. The mandrel 28 is then passed through a predrilled hole 14 of a bone plate 10 or an intramedullary nail 11 or other work piece. The end of the mandrel which is passed through the hole 14 includes tapered portions 30 and a right cylindrical portion 32. The diameter of portion 32 is selected so that it is not greater than the initial diameter of the predrilled hole 14 which permits the passage of the mandrel through the hole but is larger than the internal diameter of sleeve 26. Following passage of the mandrel through the hole, the sleeve 26 is inserted into the hole 14 which thereby reduces the effective diameter of the hole 14 to the inner diameter of the sleeve 26. At that point, the mandrel 28 is retracted in the direction of arrow 34 which causes uniform plastic deformation around the circumference of the hole 14. Preferably, the sleeve 26 is manufactured of a material having a higher modulus and yield strength than the material of the work piece. In this manner, fixation holes or other holes of orthopaedic implants may be cold worked or plastically deformed as described herein. In the case of an intramedullary nail 11 or other implant having two or more holes in close proximity to one end of the implant, it may be desirable to employ a device as shown in Figure 3 which permits the cold working of multiple fixation holes in the same operation.

Orthopaedic trauma implants are frequently manufactured of biocompatible austenitic stainless steel, preferably type 316L. However, such bone implants may be made of a biocompatible titanium-based alloy of a biocompatible cobalt based alloy. The cold working expansion process described herein may be practiced during surgery using prelubricated material that is biocompatible and adaptable for use in a sterile environment.

Referring still to Figure 3, the mandrel 28 may be withdrawn through the bone plate 10 or intramedullary nail 11 by a pulling apparatus 40.

Referring now to Figure 4, a graph of tensile loading versus strain for a generic material is shown. An elastic relationship is shown by material 100 in part A of the graph. In this zone, the strain will return to zero once the load is relieved. In zone B, however, a plastic relationship is defined. That is, once the material exceeds it elastic limit, it is permanently deformed. If the load is relieved, the strain will not return to zero but to a strain as shown by line 200. However, if the stress exceeds the ultimate tensile limit (U.T.L.), the material will exhibit excess strain and may crack or break. In performing a cold working operation in accordance with the present invention, the region surrounding the fixation hole or other hole in the work piece is plastically deformed, as represented by zone B in Figure 4. In other words, the material must be deformed enough to sufficiently exceed the elastic limit and plastically deform.

It has been noted that cold working the fastener hole 14 in a bone plate or an intramedullary nail creates a zone of residual compressive stresses around the periphery of the hole. Typically, these residual stresses will exceed two-thirds of the tensile yield stress of a material and extend radially outward past the edge of the hole by a distance equal to at least the radius of the hole. Thus, these induced residual compressive stresses decrease the peak tensile stresses experienced at the hole region during fatigue loading. This improves the life of the bone plate since it is the cyclic loading between no stress and the peak tensile stress which creates fatigue failures. In other words, the residual compressive stresses induced by cold working effectively reduces the maximum tensile fatigue stress that the bone plate will experience and thereby increases its fatigue resistance. The region where the induced residual compressive stresses lie has been referred to as the "zone of influence".

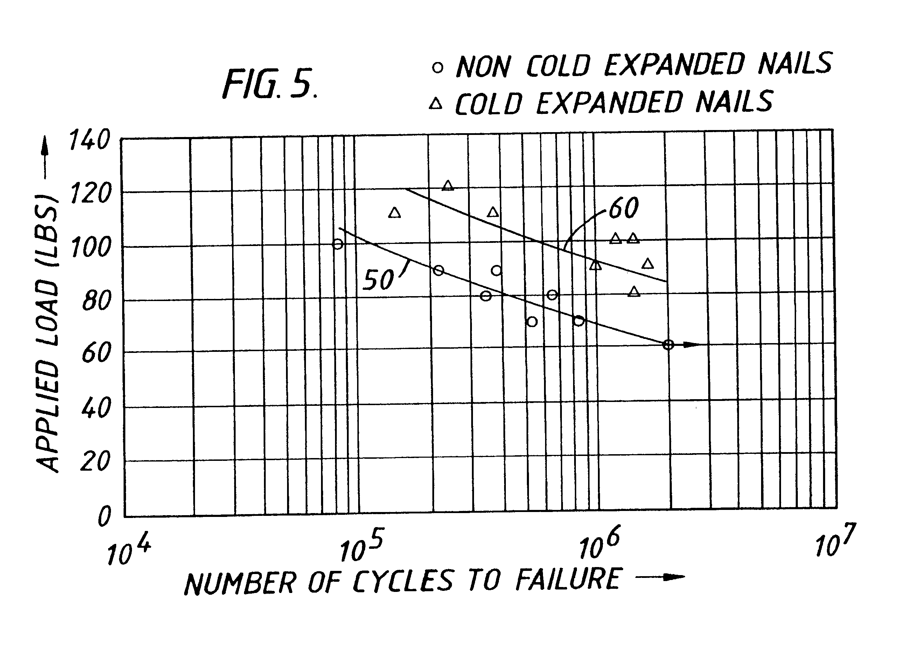

Figure 5 illustrates the results of experiments conducted to illustrate the improved performance of an orthopaedic implant manufactured in accordance with the present invention. For these experiments, 13 millimeter nominal size (ASTM F339-71) Russell-Taylor interlocking femoral intramedullary nails made of type 316L stainless steel were selected. A total of sixteen samples were fatigue tested under cyclic torsional loading. Eight samples were tested without practicing the present invention and the results of those eight tests are designated as "Non-cold Expanded Nails" in Figure 5 and represented by the circular symbol. The eight other samples were tested having been manufactured in accordance with the present invention and the results of those eight tests are designated as "Cold Expanded Nails" in Figure 5 and represented by the triangular symbol. Lines 50 and 60 in Figure 5 represent the average of the data and illustrate the average performance differences between "non-cold expanded nails" and "cold expanded nails", respectively.

Each test specimen had a transverse hole 5.12mm = 0.7mm/-0.0mm (0.209 + 0.003/-0.000" diameter) at its midshaft location. In the case of the non-cold expanded nails, these holes were drilled to a final size as noted. In the case of the cold-expanded samples, the initial drilled hole was between 4.598mm and 5.047mm (0.204 and 0.206"). Thse holes were then cold-expanded in accordance with the present invention as described above to a final diameter as noted initially above. All nails were then final polished prior to testing.

Before testing, the ends of each nail were cut off to provide an 18cm center section which included the transverse hole. Each nail section was then secured between two drill chucks providing a working length of 14.5cm. Cyclic loading was then introduced to one of the drill chucks using a R-ratio (minimum loading/maximum loading) of 0.1. Each specimen was run to failure or until a cyclic life of two million cycles was reached. As noted above, an average expansion of 4.5% was applied to the intitial starting hole for the nails which were cold-expanded and an average 2.94% expansion was retained in the hole region. None of these nails exhibited any apparent dimensional deformation at the hole region following cold expansion.

Table 1 below illustrates the final results of each of the eight non-cold expanded nails and the cold-expanded nails illustrating applied load and cycles to failure. The results from Table 1 have been plotted on Figure 5.

## TABLE 1

### RESULTS OF FATIGUE TESTS

| Nail Number | Maximum Applied Load Kg (lb) | | Cycles to Failure |
|---|---|---|---|
| Non-cold Exanded | | | |
| NCX 1 | 45.4 | (100) | 82,200 |
| NCX 6 | 40.86 | (90) | 219,140 |
| NCX 8 | 40.86 | (90) | 387,160 |
| NCX 5 | 36.32 | (80) | 344,450 |
| NCX 7 | 36.32 | (80) | 666,620 |
| NCX 2 | 31.78 | (70) | 540,980 |
| NCX 4 | 31.78 | (70) | 847,846 |
| NCX 3 | 27.24 | (60) | $2 \times 10^6$ (runout) |
| Cold Expanded | | | |
| CX 2 | 54.48 | (120) | 250,500 |
| CX 5 | 49.94 | (110) | 154,180 |
| CX 7 | 49.94 | (110) | 375,260 |
| CX 3 | 45.4 | (100) | 1,295,430 |
| CX 6 | 45.4 | (100) | 1,445,450 |
| CX 1 | 40.86 | (90) | 1,015,860 |
| CX 4 | 40.86 | (90) | 1,774,210 |
| CX 8 | 36.32 | (80) | 1,587,400 |

Referring to lines 50 and 60 of figure 5, the performance at an applied load of 45.4Kg (100 pounds) illustrates an improved performance using the present invention from approximately 120,000 cycles to failure to over 500,000 cycles to failure. This represents an improvement of over four fold. At a reduced applied load of 36.32Kg (80 pounds) the performance of the implant increased from a cycle life of approximately 440,000 to about 3,000,000 cycles, estimated by extrapolating line 60 out. The performance of the intramedullary nails tailored in accordane with the present invention indicates a substantial increase in their stability to resist fatigue failures.

Based on the results of the tests conducted, the preferred range for cold work expansion is between 2.5% to 60% of its initial diameter and most preferably between 3.5% to 4.5% of its initial diameter. It may be advantageous as well following completion of the cold-work expansion operation to machine the fixation hole to a final diameter. This may be helpful in reducing the zone of influence created by the cold working, particularly where the width of the work piece may be adversely affected by the size of the zone of influence. In that case, it may be preferably to drill a smaller initial hole, cold work that hole a predetermined amount and then machine the hole to a final diameter. This process may be used to eliminate up to one-half the width of the zone of influence.

## Claims

1. An improved orthopaedic implant comprising:
   a biocompatible base member said base member having at least one fixation hole; and
   said fixation hole having been expanded beyond the elastic limit of the implant material.

2. An implant according to claim 1 wherein the base member is made of an austenitic stainless steel, a titanium based alloy or a cobalt based alloy.

3. An implant according to claim 1 or claim 2, wherein said fixation hole is expanded at least 2.5% of its initial diameter.

4. An implant according to claim 3, wherein said fixation hole is expanded between about 2.5% to 6% of its initial diameter.

5. An implant according to claim 3, wherein said fixation hole is expanded preferably between about 3.5% to 4.5% of its initial diameter.

6. An implant according to any one of claims 1 to 5, wherein said fixation hole has been machined to a final diameter following the cold working of said fixation hole.

7. An implant according to any one of claims 1 to 6, wherein said base member being manufactured from type 316L stainless steel.

8. A method for improving the integrity of holes in orthopaedic implants comprising the steps of:
   providing an implant having at least one hole; and
   expanding the diameter of the hole beyond the elastic limit of the implant material.

9. The method of claim 8 , wherein said hole is expanded at least 2.5% of its initial diameter.

10. The method of claim 8 or claim 9, wherein said hole is expanded between 2.5% to 6% of its initial diameter.

11. The method of claim 10, wherein said hole is expanded preferably between about 3.5% to 4.5% of its initial diameter.

12. The method of any one of the preceding claims wherein the step of expanding said hole comprises the step of:
    positioning a prelubricated sleeve onto a mandrel wherein said sleeve has a modulus of elasticity and yield strength higher than said implant;
    inserting said mandrel and sleeve within said hole; and
    displacing said mandrel relative to said sleeve to expand the diameter of said hole.

13. A method according to any one of the preceding claims wherein the implant is formed from a biocompatible austentitic stainless steel, a biocompatible titanium-based alloy or a biocompatible cobalt-based alloy.

14. A method according to any one of the preceding claims comprising the step of machining the diameter of the fixation hole to a predetermined final diameter following completion of said expanding step.

# FIG.1.

FIG. 2.

# FIG. 3.

# FIG. 4.

FIG. 5.

o NON COLD EXPANDED NAILS
△ COLD EXPANDED NAILS

European Patent Office

**EUROPEAN SEARCH REPORT**

Application Number

EP 91 30 1489

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | BIOMEDIZINISCHE TECHNIK, vol. 32, no. 12, December 1987, pages 305-307; M. BÖRNER: "Ein Verriegelungsnagel mit verbesserter Ermüdungsfestigkeit" <br> * Page 306, right-hand column - page 307, left-hand column * <br> --- | 1,3-6,8 -11 | A 61 B 17/58 |
| A | DE-A-3 433 105 (KERNFORSCHUNGSZENTRUM KARLSRUHE) <br> * Page 5, line 22 - page 6, line 10; figures 1,2 * <br> --- | 1,8 | |
| A | ENGINEERING IN MEDICINE, vol. 2, no. 3, July 1973, pages 51-57; J.T. SCALES: "Ten years of fixation of fractures" <br> * The whole document * <br> --- | 1,2,7, 13 | |
| A | US-A-4 471 643 (CHAMPOUX) <br> * Column 4, line 28 - column 6, line 55; figures 1-6 * <br> --- | 1,8,12 | |
| A | MACHINE DESIGN, vol. 49, no. 28, 8th December 1977, pages 186-187; TECH BRIEFS: "Coldworking boosts fatique life of fasterner holes" <br> * The whole document * <br> --- | | **TECHNICAL FIELDS SEARCHED (Int. Cl.5)** <br><br> A 61 B <br> F 16 B <br> B 23 P |
| A | ALUMINIUM, vol. 61, no. 10, 1985, pages 746-752; L. SCHWARMANN et al.: "Lebensdauererhöhung genieteter Fügeverbindungen durch Aufdornen der Nietbohrungen" <br> * Pages 746-752 * <br> ----- | 1-14 | |

The present search report has been drawn up for all claims

| Place of search | Date of completion of the search | Examiner |
|---|---|---|
| THE HAGUE | 06-06-1991 | SCHMIERER U.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)